# EUROPEAN PATENT SPECIFICATION

(11) **EP 1 365 017 B1**
(45) Date of publication and mention of the grant of the patent: **06.09.2006**
(21) Application number: 03253257.4
(22) Date of filing: 23.05.2003
(51) Int. Cl.: C12N 5/00, C12M 3/00

(54) **Cell-culturing device and sorting method using same**
Zellkultivierungsvorrichtung und diese benutzende Verfahren zur Zellsortierung
Dispositif pour la culture de cellules et méthode de tri utilisant celui-ci

(30) Priority: 24.05.2002 JP 2002150623
(43) Date of publication of application: 26.11.2003
(73) Proprietor: National Institute of Advanced Industrial Science and Technology, Tokyo 100-8921 (JP)
(72) Inventor: Sumaru, Kimio, National Inst. of Advanced Ind., 1-1, Higashi, Tsukuba, Ibaraki 305-8565 (JP); Kameda, Mitsuyoshi, Nat. Inst. of Advanced Indust., 1-1, Higashi, Tsukuba, Ibaraki 305-8565 (JP); Kanamori, Toshiyuki, Nat. Inst. of Advanced Ind., 1-1, Higashi, Tsukuba, Ibaraki 305-8565 (JP); Shinbo, Toshio, National Inst. of Advanced Ind., 1-1, Higashi, Tsukuba, Ibaraki 305-8565 (JP)
(74) Representative: Daniels, Jeffrey Nicholas

(56) References cited:
- EP-A- 0 382 214
- WO-A-01/68799
- NICOLAU DAN V ET AL: "Patterning neuronal and glia cells on light-assisted functionalised photoresists." BIOSENSORS & BIOELECTRONICS, vol. 14, no. 3, 15 March 1999 (1999-03-15), pages 317-325, XP002251933 ISSN: 0956-5663
- NAKAYAMA YASUHIDE ET AL: "Photocontrol of cell adhesion and proliferation by a photoinduced cationic polymer surface." PHOTOCHEMISTRY AND PHOTOBIOLOGY, vol. 77, no. 5, 20 May 2003 (2003-05-20), pages 480-486, XP008020833 ISSN: 0031-8655
- NICOLAU D.V. ET AL BIOSENSORS & BIOELECTRONICS vol. 11, no. 12, 1996, pages 1237 - 1252, XP000869950

## Description

### FIELD OF THE INVENTION

The present invention relates to a photo-responsive composition having properties such that cells adhere to the composition but are released from the composition upon light irradiation of the composition, a cell-culturing device comprising the photo-responsive composition, a method of cell culturing/sorting using the device and an apparatus for use with the device.

### BACKGROUND OF THE INVENTION

Heretofore, there have been known various cell-sorting technologies, such as flow cytometry (FACS) systems and magnetic cell separating systems (MACS), which have been put to practical use. While such systems are primarily used in the sorting/collection of suspended cells, such as leucocytes or lymphocytes, they may also be used in the sorting/collection of anchorage-dependent cells if the cells are first released from the substrate on which they are being grown.

Anchorage-dependent cells attached to a substrate can easily be released through enzyme treatment using trypsin or the like. However, membrane proteins necessary for proper cell function can be undesirably degraded during the enzyme treatment, together with cell-adhesion molecules, resulting in decreased usability of the collected cells. In addition, use of flow cytometry systems can result in serious damage to cells during the process of physically separating the cells with an ultrasonic nozzle.

**[0003a]** Nicholau *et al.,* (Biosensors & Bioelectronics Vol. 11. No. 12, pp. 1237 - 1252 (1996)) discloses control of the neuronal cell attachment by functionality manipulation of diazo-naphtho-quinone/novolak photoresist surface.

**[0003b]** Nicholau *et al.,* (Biosensors & Bioelectronics Vol. 14. No. 3, pp. 317 - 325 (1999)) discloses patterning neuronal and glia cells on light-assisted functionalised photoresists.

Other technologies for releasing cultured anchorage-dependent cells have been reported. One is a technique of releasing/collecting cultured anchorage-dependent cells in the form of a sheet while maintaining the organ-specific functions of the cells by not degrading cell-adhesion substances and membrane proteins (Kikuchi et al., *J. Biomater. Sci., Polym. Edn.,* 9, 1331 (1998)). Another is a technique of patterning an adhesive substrate on a culture vessel using a microfabrication process, such as electron lithography, and then introducing bioactive substances or artificial compounds into the adhesive substrate to analyze conditions of cell adhesion and influences on biological activities (Ito, *Protein, Nucleic Acid and Enzyme,* 45, 727 (2000)).

There are also problems with these techniques however. For example, in the technique of releasing/collecting cultured anchorage-dependent cells in the form of a sheet, adhesiveness of the cells to the substrate is controlled by changing temperature. This is also the case when the cells are cultured on the bed material disclosed in EP0382214A. However, this technique is problematic in that when only a specific cell or cell population (colony) is selected for collection from various kinds of cells or cell populations in a culture vessel, it is extremely difficult to control the attaching/detaching of the specific selected cells or cell populations and not those located nearby in the culture device.

As to the technique of patterning of an adhesive substrate on a culture device, while some changes in adhesiveness of the adhesive substrate with cells are reported, such changes are caused by introducing the bioactive substances in the substrate, and the adhesiveness of cells to the substrate cannot be flexibly controlled without such substances.

For these reasons, in the technology of sorting/collecting cultured anchorage-dependent cells, accurate separation of cells, while maintaining the organ-specific functions of the separated cells, has not been achieved.

### SUMMARY OF THE INVENTION

In view of the above circumstances, it is therefore an objective of the present invention to provide a device and a method for culturing and sortingly collecting cells, particularly anchorage-dependent cells, through a simple operation while maintaining respective organ-specific functions of the cells without damage to the cells.

As a result of continuous research into the above objective, the inventors identified a photo-responsive composition, having the property of differential adhesiveness upon light irradiation, that can be used as an adhesive surface for the growth of anchorage-dependent cells in a culture device, and that can be used to easily regulate the attaching/detaching of cultured cells or cell populations from the culture device, while maintaining the respective organ-specific functions of the cells. Based on this knowledge, the inventors have finally accomplished the present invention.

Specifically, according to a first aspect of the present invention, there is provided a cell-culturing device, said device comprising a culturing dish having a photo-responsive composition thereon, wherein said photo-responsive composition comprises a photo-responsive material having a property of differential adhesiveness for a cell based on an amount of light irradiation to which said photo-responsive composition is exposed, wherein the property of differential adhesiveness is capable of regulating attaching and detaching of a cell existing on said device.

In the photo-responsive composition set forth in the first aspect of the present invention, the light irradiation-induced properties of the composition may be reversible in the absence of light irradiation, and the ability of the composition to cycle between having the properties induced by light irradiation and returning to an uninduced state may be unlimited.

The photo-responsive composition is extremely flexible in that it may have a number of different light irradiation-induced properties. For example, the adhesiveness of the photo-responsive composition may decrease under light irradiation, and be recovered in the absence of light or a decrease in the amount or intensity of the light. Alternatively, the adhesiveness of the photo-responsive composition may increase under light irradiation, and be decreased in the absence of light or a decrease in the amount or intensity of the light. The adhesiveness of the photo-responsive composition may also be increased or decreased when exposed to a particular wavelength (or wavelength range) of light, and returned to its previous state in the absence of the particular wavelength.

The adhesiveness of the photo-responsive composition changed by the exposure of the photo-responsive composition to a particular wavelength of light may be held stably in the dark until further exposure to the same wavelength of light increases the change or another wavelength of light reverses the change.

Further, the change in the property of differential adhesiveness may include at least a reduction or increase in adhesiveness to cells.

The cell-culturing device may be formed as a chamber.

According to a second aspect of the present invention, there is provided a method of growing and collecting a cell, the method comprising the steps of growing a cell in the cell-culturing device of the first aspect of the present invention, irradiating the photoresponsive composition of the cell-culturing device with light to release the cell attached to the photo-responsive composition; and collecting the released cell. In a related aspect, the cell-culturing device may be used to simply grow the cell, without collecting the cell from the device.

According to the present invention, there is provided a method of collecting a cell from a cell-culturing device of the first aspect of the present invention, the method comprising the steps of irradiating the photo-responsive composition of the cell-culturing device with light to release a cell attached to the photo-responsive composition; and collecting the released cell.

According to the present invention, there is provided a method of selectively collecting a cell from a cell-culturing device of the first aspect of the present invention, the method comprising the steps of: irradiating the photo-responsive composition of the cell-culturing device with light, wherein the light is irradiated onto a selected region of the photo-responsive composition to release only a cell attached to the selected region; and sortingly collecting the released cell.

In the method set forth above, the irradiating step may further include physically stimulating the cell-culturing device and/or supplying an eluate into the cell-culturing device. Furthermore, in each of these aspects of the present invention, preferably the cell is an anchorage-dependent cell.

According to a third aspect of the present invention, there is provided a cell culturing/sorting apparatus comprising a cell-culturing device of the first aspect of the present invention, a means for supplying a culture solution into the device, a means for selectively irradiating a given position on the photo-responsive composition of the device with light, a means for detecting respective positions of the cells on the photo-responsive composition, and a means for sorting the cells released from the photo-responsive composition through the light irradiation.

This apparatus may further include a means for physically stimulating the cell culturing device and/or a means for supplying an eluate into the cell-culturing device.

**[0022a]** According to a fourth aspect of the present invention, there is provided a photo-responsive material, wherein said material comprises a thermo-sensitive polymer having both hydrophilic and hydrophobic properties that change in response to a temperature change, and wherein said polymer is modified by a photochromic dye, wherein said photo-responsive material exhibits differential adhesiveness for a cell based on an amount of light irradiation to which said photo-responsive material is exposed, such that the property of differential adhesiveness is capable of regulating attaching and detaching of a cell existing on a cell-culturing device.

### BRIEF DESCRIPTION OF THE DRAWINGS

FIG. 1 is a schematic flow chart showing a cell culturing/sorting method of the present invention.

FIG. 2 is a schematic flow chart showing a cell sorting method of the present invention in which different kinds of cells are sortingly collected.

FIG. 3 is a schematic diagram showing one modification of the culturing/sorting method according to the present invention.

FIG. 4 is a block diagram showing a cell culturing/sorting apparatus according to one embodiment of the present invention.

FIG. 5 is an explanatory block diagram showing a selective cell releasing process in a cell culturing device of the cell culturing/sorting apparatus in FIG. 4.

### DESCRIPTION OF THE PREFERRED EMBODIMENT

The term "photo-responsive composition" herein means a composition having at least a surface that is adhesive to cells under particular conditions of light irradiation and that may serves as an anchorage area for an anchorage-dependent cell or a surface onto which a cell is attached. In the present invention, the photo-responsive composition is made of a photo-responsive material having at least a property of differential adhesiveness with cells in response to changes in light irradiation, including changes in duration, intensity and wavelength. For example, the photo-responsive material for forming the photo-responsive composition may be (1) a material capable of converting light energy into heat to provide a locally increased temperature that in turn causes changes in the surface properties of the photo-responsive composition, (2) a material having a composition such that the oxidation state of the surface of the photo-responsive composition may change in response to light irradiation, or (3) a material having a composition such that the structure of the material may be isomerized in response light irradiation, which in turn leads to changes in polarizability and/or hydrophilic-hydrophobic properties of the photo-responsive composition.

Preferably, the light irradiation-induced properties of the photo-responsive composition of the present invention are reversible in the absence of light irradiation, and the ability of the photo-responsive composition to cycle between having the properties induced by light irradiation and returning to an uninduced state is unlimited. This cycling ability is termed herein as "differential adhesiveness."

It should be understood that the photo-responsive composition also includes compositions (a) whose adhesiveness increases in the response to changes in the duration, intensity or wavelength of light, (b) whose adhesiveness decreases in the response to changes in the duration, intensity or wavelength of light, and (c) that the changes in adhesiveness can be temporary, reversible or permanent.

More specifically, the above photo-responsive material (1) may comprise a photo-responsive material prepared by bonding a dye or pigment molecule capable of converting light into heat (a dye or pigment generally has a property capable of converting light to heat) to a thermo-sensitive polymer having hydrophilic-hydrophobic properties that change in response to a temperature change, such as poly (N-isopropylacrylamide) and polyvinylether. In this case, the dye or pigment molecule and the thermo-sensitive polymer may be polymerized on the substrate surface of a culturing device.

Preferably, the dye or pigment is a material that absorbs light and contains organic dyes, mineral pigments and carbons. Preferred examples include Indigo, quinacridones, porphyrins, Fe₂O₃, HgS, CoO·nAl₂O₃ and carbon black.

Preferably, the thermo-sensitive polymer having hydrophilic-hydrophobic properties is one of the following: a homopolymer or copolymer composed of N-isopropylacrylamide, vinylmethylether, dimethylaminoethylmethacrylate, oxyethylenevinylethers, sodium styrenesulfonate and/or vinylbenzyltrimetylammonium chloride.

The dye or pigment and the thermo-sensitive polymer may be bonded together by covalent bonding, hydrogen bonding, ionic bonding and/or hydrophobic bonding.

Preferably, the area density of the dye or pigment in the photo-responsive material is from about 10⁻⁹ mol/cm² to about 10⁻³ mol/cm², more preferably from about 10⁻⁸ mol/cm² to about 10⁻⁴ mol/cm², most preferably from about 10⁻⁷ mol/cm² to about 10⁻⁵ mol/cm², and the optical density of the dye or pigment in the effective range of wavelength is from about 0.01 to about 4, more preferably from about 0.05 to about 2, most preferably from about 0.1 to about 1.

The polymerization of the dye or pigment molecule and the thermo-sensitive polymer to the substrate surface of a culturing device may be performed by any suitable method, including surface graft polymerization initiated by plasma irradiation or an electron bombing, and an ion complex method.

Preferably, the thickness of the photo-responsive composition comprising the dye or pigment molecule and the thermo-sensitive polymer on the culture dish is from about 0.001 µm to about 5 µm, more preferably from about 0.005 µm to about 1 µm, most preferably from about 0.01 µm to about 0.1 µm.

The above photo-responsive material (2) may be a photocatalyst material such as titanium oxide. The photocatalyst material may be used directly as the photo-responsive composition to form a photocatalyst layer on the substrate surface of a culturing dish through a sintering method or the like.

In addition to titanium oxide, the photocatalyst material may also be a photo-catatytic metal oxide such as VO₂, WO₃, and MoO₃.

Preferably, the thickness of the photo-responsive composition comprising the photocatalyst material on the culture dish is from about 0.001 µm to about 5 µm, more preferably from about 0.005 µm to about 1 µm, most preferably from about 0.01 µm to about 0.1 µm.

The photo-responsive material (3) may include: a photo-responsive molecule that is used directly as the photo-responsive composition, such as an organic compound derivative, for example, azobenzene, diarylethen, spiropyran, spirooxazine, fulgide or leuko chromophore; a monomer prepared by bonding the photo-responsive molecules themselves; and a polymer prepared by polymerizing the monomers.

When a photo-responsive molecule itself is used directly as the photo-responsive composition, it may be chemically bonded to the substrate surface of a culturing dish by using a silane coupling agent or the like, or physically bonded thereto through hydrophobic interaction. When the monomer is used, it may be polymerized on the substrate surface of a culturing dish. When the polymer is used, it may be dissolved in a suitable solvent, and the obtained solution may be applied on the substrate surface of a culturing dish.

In addition to the photo-responsive molecule in the photo-responsive composition, other components may be included in the photo-responsive composition such as homopolymers or copolymers composed of N-isopropylacrylamide, vinylmethylether, dimethylaminoethylmethacrylate, oxyethylenevinylethers, sodium styrenesulfonate and/or vinylbenzyltrimetylammonium chloride.

Introduction of the photo-responsive composition comprising the photo-responsive molecule to the substrate surface of a culturing dish may be performed by spin coating, physical adsorption, silane coupling and gold-tiol method.

Polymerization of the photo-responsive composition comprising the photo-responsive molecule to the substrate surface of a culturing dish may be performed by surface graft polymerization initiated by plasma irradiation or electron bombing, and ion complex method.

Suitable solvents for dissolving the polymers include water, alcohols, esters, aromatic solvents, ketones, ethers (including cyclic ethers such as THF and dioxane), haloparaffins, and DMSO.

Preferably, the thickness of the photo-responsive composition comprising the photo-responsive molecule on the culture dish is from about 0.001 µm to about 5 µm, more preferably from about 0.005 µm to about 1 µm, most preferably from about 0.01 µm to about 0.1 µm.

The photo-responsive composition made of one or more of the above photo-responsive materials has a property of releasing a cell attached thereto in response to light irradiation. Even if the cell is not completely released through the light irradiation, the adhesiveness of the composition is sufficiently reduced, and the cell can be readily released by adding an eluate such as a diluted solution of protease or a salt solution, or by applying physical stimulation such as vibration. In the present invention, no amount or an ineffective amount of an enzyme such as trypsin needs to be used to release cells from the surface of the cell-culturing device. This feature provides a significant advantage of allowing a cell to be released while adequately maintaining the integrity of cell-adhesion molecules, such as ECM (extracellular matrix) and membrane proteins, which play important roles in expressing and retaining cell functions.

The photo-responsive composition of the present invention may be comprised of only one of the photo-responsive materials (1), (2) and (3) discussed above. The photo-responsive composition of the present invention may optionally be comprised of any combination of one or more of each or any of the three types.

The cell-culturing device of the present invention comprises a culturing dish with the photo-responsive composition formed on the substrate surface of the culturing dish that serves as the cell receiving space.

The culturing dish may be constructed from materials commonly used to make culturing dishes, including glass, polystyrene, polyvinyl chloride, polymethylpenthene, polyethylene telephthalate, polyethylene, and polypropylene.

The cell-culturing device of the present invention may be a component of a cell-culturing chamber. The cell-culturing chamber is a chamber that is used to maintain temperature, humidity and the concentration of particular gases such as O₂ and CO₂ to which the cells in the culturing dishes are exposed. The cell-culturing chamber may have other components as well, such as a system for supplying cell culture media to the cell-culturing device, a system for removing cell culture media from the cell-culture device, and a system for identifying the position of a particular cell or cell population in the cell-culturing device.

In a preferred embodiment of the photo-responsive composition of the present invention comprising the photo-responsive material (1) above, the photo-responsive material (1) comprises a thermosensitive copolymer comprising 20% sodium styrene sulfate, 20% of vinylbenzyl trimethylammonium chloride and 60% of N, N'-methylene-bis-acrylamide, wherein the thickness of the photo-responsive compositions is about 0.1 µm, applied to a thin layer of carbon black, and in a preferred embodiment of the cell-culturing device, the photo-responsive composition so produced is applied to the substrate surface of a culture dish.

In a preferred embodiment of the photo-responsive composition of the present invention comprising the photo-responsive material (2) above, the photo-responsive material (2) comprises titanium oxide as the photocatalyst material, and in a preferred embodiment of the cell-culturing device, the photo-responsive composition is applied to the substrate surface of a culture dish, where in the thickness of the photo-responsive compositions is about 1 µm.

In a preferred embodiment of the photo-responsive composition of the present invention comprising the photo-responsive material (3) above, the photo-responsive material (3) comprises a photo-responsive copolymer comprising 5% of a vinyl compound containing nitrospiropyran and 95% N-isopropylacrylamide, and in a preferred embodiment of the cell-culturing device, the photo-responsive composition is applied to the substrate surface of a culture dish, where in the thickness of the photo-responsive compositions is about 0.1 µm.

With reference to the drawings, a cell culturing/sorting method using the above cell-culturing device will now be described.

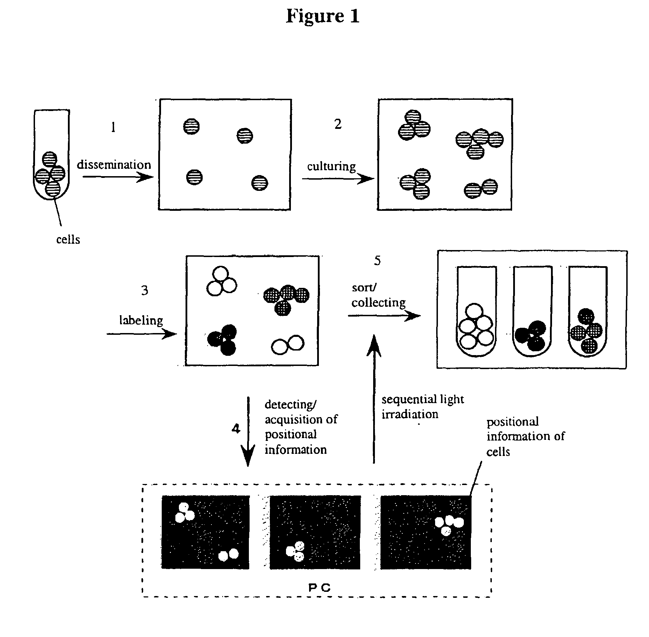

FIG. 1 is a schematic flow chart showing a cell culturing/sorting method of the present invention.

A cell culturing solution is put into a cell-culturing device of the present invention to disseminate/culture/proliferate a sample containing, for example, a plurality of cells types (1, 2). The cells attach to the photo-response composition comprising the photo-responsive material in the cell-culturing device, and proliferate, using the photo-response composition as an anchorage. A polyclonal or monoclonal antibody that specifically recognizes and binds to an epitope expressed by one of the plurality of cell types, which is labeled with, for example, a fluorescent dye, is added to the cell-culturing device, and allowed to bind to the cells. In this process, one type of antibody may be used corresponding to one cell type. Alternatively, a plurality of antibodies may be used corresponding to respective cell types, and the antibodies may be labeled with different fluorescent dyes. The added antibody makes it possible to detect the positional information of the cell or cell colony to be sorted (3). Then, the cell or cell colony to be sorted in the cell-culturing device is detected by a color CCD camera, and the detected positional information is transferred to a personal computer (PC) (4). Based on the positional information, light is irradiated on the position of the target cell or cell colony. The light irradiation causes a change in the adhesive properties of the photo-responsive composition, or a reduction in adhesiveness of the photo-responsive composition, where the cell or cell colony is located. Thus, the target cell or cell colony can be selectively released from the surface of the cell-culturing device, and sortingly collected (5).

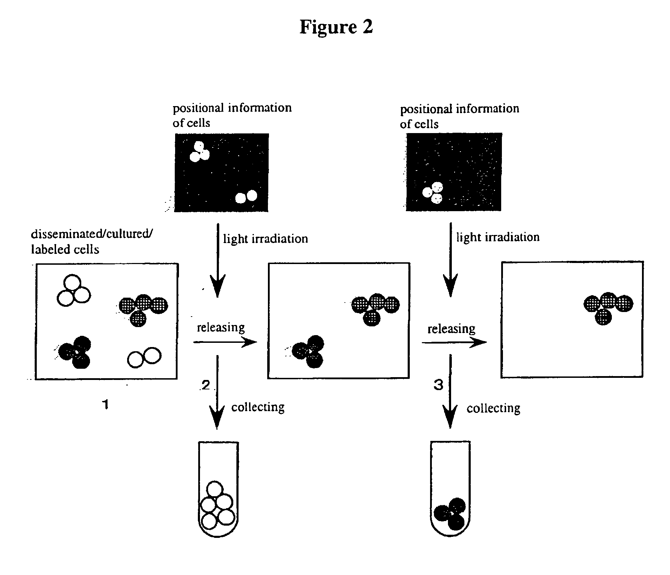

FIG. 2 is a flow chart showing a cell sorting method of the present invention in which a plurality of cell types are each sortingly collected. For sortingly collecting a plurality of cell types, a plurality of differently labeled antibodies, for example each with a different fluorescent dye, are used that correspond to each respective cell type. Each different antibody specifically recognizes and binds to an epitope unique to one of the plurality of cell types (1). The positional information of each of the cell types is obtained based on differences in fluorescence of the fluorescent dyes, and then light is locally irradiated on the position of the cell generating a specific fluorescence to release that one cell type or cell colony located at the position, and then the released cell or cell colony is collected (2). Then, light is irradiated on another position where a second type of cell or cell colony to be subsequently collected is located, thus releasing the second cell or cell colony which is then collected (3). The above process can be repeated to sortingly collect the cells for each of the plurality of cell types.

While a plurality of cell types are sorted, respectively, in the above description, the present invention is not limited to this process. For example, light may be irradiated over the entire surface of the cell-culturing device to release all of the cells attached to the photo-responsive composition and the cells then collected. This method is particularly useful when a large amount of one kind of cell is to be cultured and collected.

In the present invention, various fluorescent labeling methods may be used as well as the above method of using fluorescent dye. For example, a polynucleotide encoding an enzyme constituting a luminous system, such as luciferase, may be introduced into a cell. Further, for sorting cells having different morphologies, a target cell may be sortingly collected through light irradiation under microscopic observation, without particular fluorescence labeling.

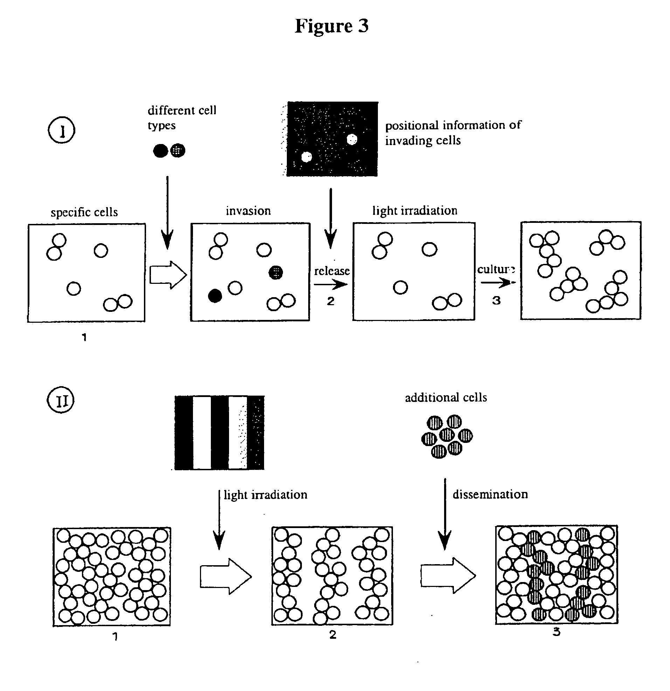

With reference to FIG. 3, one modification of the aforementioned culturing/sorting method will be described below.

In FIG. 3, a flow chart (I) shows a process of cultivating a pure culture of only a specific cell type.

In order to cultivate a pure culture of only a specific cell type, the photo-responsive composition of the present invention is formed, for example, on the aforementioned culture dish, and the specific cell type is cultured in the device (1). If another cell type invades therein during culturing, light can be sequentially irradiated on the positions of the invading cells to release and remove the invading cells (2), and only the desired cell type will be left on the photo-responsive composition and continuously cultured (3). For identifying the invading cells, the specific cell type to be cultivated as a pure culture may be labeled with fluorescence to allow non-fluorescent-labeled cells to be identified as the invading cells. Alternatively, if the invading cells can be morphologically identified, the invaded cells can be released and removed through light irradiation under microscopic observation, without particular fluorescence labeling, in the same manner as described above.

In FIG. 3, a flow chart (II) shows a process of patterning cells and co-culturing a plurality of patterned cells.

For patterning cells, a certain cell type is cultured and proliferated until the cells spread over the photo-responsive composition (1). Then, according to a predetermined pattern, light is irradiated on one or more specified regions of the cell-culturing device to remove the cells located in the specified regions. The remaining cells form the predetermined pattern (2). Then, another cell type is disseminated in the specific regions devoid of cells resulting in a plurality of cell types distinctively patterned (3). Alternatively, the patterning of cells may be achieved by periodically irradiating a specified region with light while culturing the cells to prevent cells from attaching to the specified region. In this case, the cultured cells can be arranged with enhanced flexibility.

The above methods are useful in analyzing the communication between cells or in using cells to produce a bioactive substance that is produced under the coexistence of a plurality of cell types.

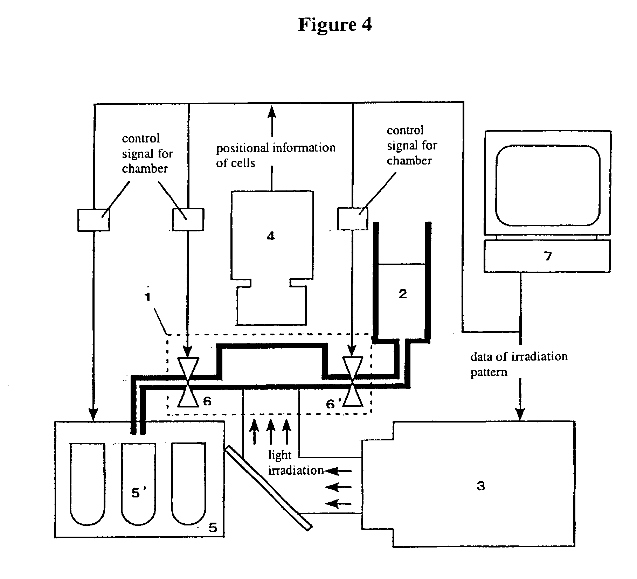

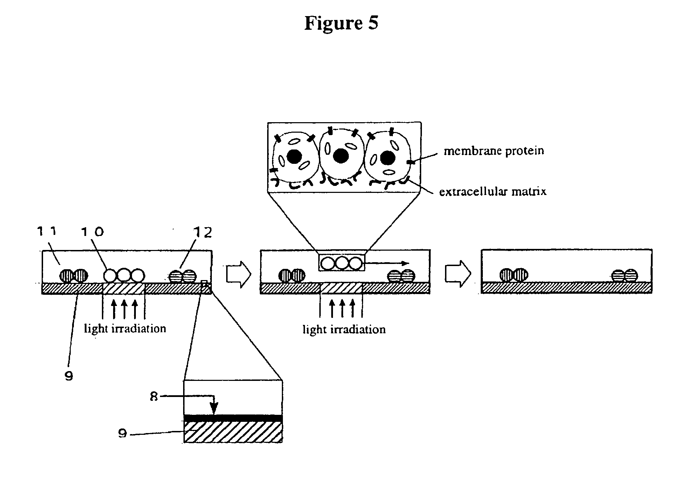

A cell culturing/sorting apparatus of the present invention comprises a cell-culturing device including a photo-responsive composition which has differential adhesiveness with cells in response to light irradiation, a means for supplying a culture solution to the device, a means for irradiating any selected position of the photo-responsive composition of the device with light, a means for detecting respective positions of cells on the photo-responsive composition, and a means for sorting the cells released from the photo-responsive composition through the light irradiation. The cell culturing/sorting apparatus may further include a means for supplying an eluate according to need. With reference to FIGs. 4 and 5, the cell culturing/sorting device of the present invention will be more specifically described.

FIG. 4 is a block diagram showing a cell culturing/sorting apparatus according to one embodiment of the present invention. FIG. 5 is an explanatory block diagram showing a selective cell releasing process in a cell-culturing chamber of the cell culturing/sorting apparatus in FIG. 4.

The apparatus comprises a cell-culturing chamber (1) which includes a cell-culturing device comprising the photo-responsive composition (8), a culture reservoir for supplying a culture solution to the device (2), a projector (3) serving as a micro 2-dimensional pattern-irradiating optical system for irradiating any selected position of the photo-responsive composition (8) of the cell-culturing chamber with light, a color CCD camera (4) for detecting respective positions of cells on the photo-responsive composition (8) and transmitting a signal of the detected positional information to an after-mentioned control unit, a sortingly collecting device (5) including a plurality of switchable collecting vessels (5') to sort the cells released from the photo-responsive composition (8) through the light irradiation, switching valves (6) and (6') provided, respectively, in a first passage for providing fluid communication between the culture reservoir (2) and the cell-culturing chamber (1) and a second passage for providing fluid communication between the cell-culturing chamber and the sortingly collecting device (5), and the control unit (7) for controlling the entire operation of the apparatus. The apparatus may further include an eluate-supplying device (not shown) for supplying an eluate to the cell-culturing chamber (1) according to need.

The operation of the apparatus will be described. The switching valve (6') is first opened and closed to supply a given amount of culture solution from the culture reservoir (2) to the cell-culturing chamber (1). Then, cells are disseminated in the cell-culturing device (1), and cultured therein. Among the cells, target cells (10) may be labeled with fluorescence in advance, or may be labeled with fluorescence after culturing. The photo-responsive composition (8) of the cell-culturing chamber (1) is made of a photo-responsive material and formed on the substrate (9) of the culturing dish of the cell-culturing device. The respective positions of the cells on the photo-responsive composition (8) are detected by the color CCD camera (4). The positional information signal is entered to the control unit (7). Then, the target cells (10) to be sorted are identified according to the fluorescent label, and the projector is adjusted to irradiate the target cells (10) with light. The position to be irradiated with light from the projector light (the "light irradiation position") may be automatically adjusted by the control unit, or may be manually adjusted while observing a monitor screen.

The light irradiation locally changes the properties of the photo-responsive composition (8) or locally reduces the adhesiveness of the photo-responsive composition (8) with the target cells (10). Thus, only the target cells (10) are released, and suspended in the culture solution. Even if the cells are not completely released, the adhesiveness of the photo-responsive composition (8) is sufficiently reduced, and the cells (10) can be selectively released by opening a switching valve (not shown) of the eluate supply device to supply the eluate to the culture chamber (1). Alternatively, a device for applying a physical stimulation such as vibration may be used in combination with, or as a substitute for, the eluate supply device. The cells released through the light irradiation, and the eluate or the physical stimulation, maintain the integrity of their ECM (extracellular matrix) and membrane proteins.

Then, the switching valves (6) and (6') are opened to supply the culture solution from the culture reservoir (2) to the cell-culturing chamber (1) to collect the released cells (10) in a collecting vessel (5') of the sortingly collecting device (5).

The above process is repeated to sortingly collect other cells (11) and (12) sequentially.

In the cell culturing/sorting apparatus according to one embodiment of the present invention, the cell-culturing chamber is configured to allow the photo-responsive composition of the cell-culturing device to be irradiated with light from the outside, and to allow cells attached on the photo-responsive composition to be monitored. The micro 2-dimensional pattern irradiating optical system is operable to irradiate any selected region of the photo-responsive composition of the cell-culturing chamber with light in the spatial scale of a single cell or a cell population. Preferably, the monitor has sufficient resolution, optical power and sensitivity to identify a single cell or a cell population. When a plurality of fluorescence dye-carrying antibodies are used to identify the cell types, the cell types can be preferably identified by color. Preferably, the control unit is operable to acquire the positional information of the cultured cells based on the observation of the monitor, and to control the light irradiating from the micro 2-dimensional pattern-irradiating optical system according to the acquired positional information, and to the feed the culture solution for collecting the released cells to the chamber.

While the cell dissemination/culturing/proliferation process is performed in a cell-culturing device such as that of the cell-culturing chamber described above, the present invention can also be applied to a process of simply introducing cells to a cell-culturing device and allow them to attach to the photo-responsive composition of the device, and then sortingly collecting the cells at a chosen time. For example, this process is useful in separating a plurality of cell types contained in a tissue.

### EXAMPLE 1

10% TiO₂ hydrosol solution was casted onto a glass substrate and dried for 30 minutes at 80°C. Then it was sintered for 5 minutes at 150°C, and a layer of a photo-responsive composition with thickness of about 1 µm resulted on the surface of the glass substrate (a "photo-responsive surface"). It was fixed to the bottom of cell culture dish, and CHO (Chinese hamster ovary) cells were dispersed on the photo-responsive surface and incubated for 6 hours. When the light which activates the photo-responsive surface was irradiated at the intensity of 100mW/cm² onto the photo-responsive surface, the polygonal shape of the cells, which had been adhered to the surface and extended, changed into a round shape. Although no apparent evidence of cell exfoliation by the light irradiation was shown, this change in the shape of the cells suggested strongly the decrease in adhesive strength of the cells.

### EXAMPLE 2

The diarylethene derivative, cis-1,2-dicyano-1,2-bis (2,4,5-trimethyl-3-thienyl)ethane, which is a photochromic dye and becomes structurally isomerized and changes its absorption spec trum in response to light irradiation, was uniformly applied to a glass substrate through a spin coat method to obtain a layer of a photo-responsive composition on the surface of the glass substrate (a "photo-responsive surface"). The obtained photo-responsive surface was incorporated in a system composed of a micro 2-dimensional pattern-irradiating optical system, a monitor and a control system for controlling them. An experimental test was carried out to check whether the properties of the photo-responsive surface were reversibly controlled through light irradiation using a blue light having a wavelength band of 400 to 440 nm (intensity: 180 mW/cm²) to isomerize the photochromic dye into a colored closed-ring state and a yellow light having a wavelength band of 500 to 600 nm (intensity: 160 mW/cm²) to reverse the dye to a bleached open-ring state. As a result, it was verified that the isomerization state of the photo-responsive surface at a given position on the photo-responsive surface could be reversibly changed in a time scale of about 5 seconds with an accuracy of 30 µm resolution. The degree of isomerization was tunable continuously by controlling the intensity of light and irradiation time, and did not change in the absence of light. The isomerization property of the photo-responsive surface did not change after more than 10,000 times of iteration of isomerization.

According the present invention, cells can be separated and collected with a high degree of accuracy which has not been achieved heretofore, while maintaining the integrity and function of ECM and membrane proteins during the separation of the cells. Thus, in addition to the accurate cell separation, the respective organ-specific functions of the cells can also he maintained. As a result, the present invention provides a cell culturing/separating/collecting system significantly useful for anchorage-dependent cells. Further, the photo-responsive composition comprising a photo-responsive material having a reversibly changeable surface property makes it possible to repeat the releasing operation through light irradiation and the dissemination of different cell types so as to control the type, position and arrangement of the cultured cells on the photo-responsive composition with a high degree of accuracy.

## Claims

1. A cell-culturing device, said device comprising a culturing dish having a photo-responsive composition thereon, wherein said photo-responsive composition comprises a photo-responsive material having a property of differential adhesiveness for a cell based on an amount of light irradiation to which said photo-responsive composition is exposed, wherein the property of differential adhesiveness is capable of regulating attaching and detaching of a cell existing on said device.

2. The photo-responsive material according to claim 1, where the light irradiation is defined by intensity, duration or wavelength of the light, or a combination of these factors.

3. The cell-culturing device as defined in claim 1, wherein said culturing dish is a vessel suitable for growth of a cell comprised of a compound selected from the group consisting of glass, polystyrene, polyvinyl chloride, polymethylpenthene, polyethylene telephthalate, polyethylene and polypropylene.

4. The cell-culturing device as defined in claim 1, wherein said photo-responsive material is selected from the group consisting of (1) a composition comprising a dye or pigment, and a thermo-sensitive polymer having hydrophilic-hydrophobic properties that change in response to a temperature change, (2) a photocatalyst material, and (3) a photo-responsive molecule having the property of differential adhesiveness for a cell based on an amount of light irradiation to which it is exposed.

5. The cell-culturing device as defined in claim 4, wherein said dye or pigment is a material that absorbs light, and is selected from the group consisting of an organic dye, a mineral pigment and carbon.

6. The cell-culturing device as defined in claim 4, wherein said dye or pigment is a material that absorbs light, and is selected from the group consisting of indigo, a quinacridone, a porphyrin, Fe₂O₃, HgS, CoO.n.Al₂O₃ and carbon black.

7. The cell-culturing device as defined in claim 4, wherein said thermo-sensitive polymer is selected from the group consisting of a homopolymer and a copolymer, wherein said homopolymer and said copolymer consist of one or more of N-isopropylacrylamide, vinylmethylether, dimethylaminoethylmethacrylate, a oxyethylenevinylether, sodium styrenesulfonate and vinylbenzyltrimetylammonium chloride.

8. The cell-culturing device as defined in claim 4, wherein said photocatalyst material is selected from the group consisting of TiO₂, VO₂, WO₃ and MoO₃.

9. The cell-culturing device as defined in claim 4, wherein said photo-responsive molecule is one or more organic compounds selected from the group consisting of a azobenzene, a diarylethene, a spiropyran, a spirooxazine, a fulgide and a leuko chromophore, and wherein two or more of said photo-responsive molecules may be in the form of a functional copolymer.

10. The cell-culturing device as defined in claim 1, wherein said cell is an anchorage-dependent cell.

11. A method for culturing and collecting a cell, said method comprising:
(a) placing a cell in a cell-culturing device, said device comprising a culturing dish having a photo-responsive composition thereon, wherein said photo-responsive composition comprises a photo-responsive material having a property of differential adhesiveness for a cell based on an amount of light irradiation to which said photo-responsive composition is exposed,
(b) culturing said cell in said device for a selected time period,
(c) irradiating a selected region of the photo-responsive composition with light to release a cell attached to the photo-responsive composition at the selected region, and
(d) collecting said released cell.

12. The method as defined in claim 11, wherein said cell is an anchorage-dependent cell.

13. The method as defined in claim 11, wherein said selected region of the photo-responsive composition is the entire region covered with the photo-responsive composition.

14. The method as defined in claim 11, wherein said method further comprises physically stimulating said cell-culturing device or supplying an eluate into said cell-culturing device, or both, after said irradiating to aid in the releasing of a cell attached to the photo-responsive composition.

15. A cell culturing and sorting apparatus, said apparatus comprising:
(a) a cell-culturing device, said device comprising a culturing dish having a photo-responsive composition thereon, wherein said photo-responsive composition comprises a photo-responsive material having a property of differential adhesiveness for a cell based on an amount of light irradiation to which said photo-responsive composition is exposed;
(b) a means for supplying a culture solution into said cell-culturing device;
(c) a means for determining positional information of a cell on said photo-responsive composition;
(d) a means for irradiating a selected position of said photo-responsive composition of said cell-culturing device with light; and
(e) a means for sorting a cell released from said photo-responsive composition through said light irradiation.

16. The apparatus as defined in claim 15, which further includes a means for physically stimulating said cell-culturing device or a means for supplying an eluate into said cell-culturing device, or both.

17. A photo-responsive material, wherein said material comprises a thermo-sensitive polymer having both hydrophilic and hydrophobic properties that change in response to a temperature change, and wherein said polymer is modified by a photochromic dye, wherein said photo-responsive material exhibits differential adhesiveness for a cell based on an amount of light irradiation to which said photo-responsive material is exposed, such that the property of differential adhesiveness is capable of regulating attaching and detaching of a cell existing on a cell-culturing device.

18. The photo-responsive material according to claim 17, where the light irradiation is defined by intensity, duration or wavelength of the light, or a combination of these factors.

19. A cell-culturing device, said device comprising a culturing dish having the photo-responsive material according to claim 17 thereon.

## Patentansprüche

1. Zellkultiviervorrichtung, wobei die Vorrichtung eine Kulturschale mit einer Licht-reaktiven Zusammensetzung darauf umfasst, wobei die Licht-reaktive Zusammensetzung ein Licht-reaktives Material umfasst, das die Eigenschaft eines unterschiedlichen Haftvermögens für eine Zelle auf Grundlage der Menge der Lichtbestrahlung aufweist, gegenüber der die Licht-reaktive Zusammensetzung ausgesetzt wird, wobei die Eigenschaft des unterschiedlichen Haftvermögens dazu in der Lage ist, die Anlagerung und Ablösung einer Zelle zu regulieren, die auf dieser Vorrichtung vorhanden ist.

2. Licht-reaktives Material nach Anspruch 1, wobei die Lichtbestrahlung durch Intensität, Dauer oder Wellenlänge des Lichtes oder eine Kombination dieser Faktoren definiert ist.

3. Zellkultiviervorrichtung nach Anspruch 1, wobei die Kulturschale ein Gefäß ist, das zum Wachstum einer Zelle geeignet ist, umfassend eine Verbindung ausgewählt aus der Gruppe, die aus Glas, Polystyrol, Polyvinylchlorid, Polymethylpenten, Polyethylenterephthalat, Polyethylen und Polypropylen besteht.

4. Zellkultiviervorrichtung nach Anspruch 1, wobei das Licht-reaktive Material aus der Gruppe ausgewählt ist, die aus (1) einer Zusammensetzung, die einen Farbstoff oder ein Pigment und ein wärmeempfindliches Polymer umfasst, das hydrophile-hydrophobe Eigenschaften aufweist, die sich in Reaktion auf eine Temperaturveränderung verändern, (2) einem Photokatalysatormaterial und (3) einem Licht-reaktiven Molekül besteht, das die Eigenschaft eines unterschiedlichen Haftverinögens für eine Zelle aufweist, auf Grundlage der Menge der Lichtbestrahlung, dem es ausgesetzt wird.

5. Zellkultiviervorrichtung nach Anspruch 4, wobei der Farbstoff oder das Pigment ein Material ist, das Licht absorbiert und aus der Gruppe ausgewählt ist, die aus einem organischen Farbstoff, einem Mineralpigment und Kohlenstoff besteht.

6. Zellkultiviervorrichtung nach Anspruch 4, wobei der Farbstoff oder das Pigment ein Material ist, das Licht absorbiert und aus der Gruppe ausgewählt ist, die aus Indigo, einem Chinacridon, einem Porphyrin, Fe₂O₃, HgS, CoO.nAl₂O₃ und Kohlenschwarz besteht.

7. Zellkultiviervorrichtung nach Anspruch 4, wobei das wärmeempfindliche Polymer aus der Gruppe ausgewählt ist, die aus einem Homopolymer und einem Copolymer besteht, wobei das Homopolymer und das Copolymer aus einem oder mehreren von N-Isopropylacrylamid, Vinylmethylether, Dimethylaminoethylmethacrylat, einem Oxyethylenvinylether, Natriumstyrolsulfonat und Vinylbenzyltrimethylammoniumchlorid besteht.

8. Zellkultiviervorrichtung nach Anspruch 4, wobei das Photokatalysatormaterial aus der Gruppe ausgewählt ist, die aus TiO₂, VO₂, WO₃ und MoO₃ besteht.

9. Zellkultiviervorrichtung nach Anspruch 4, wobei das lichtempfindliche Molekül ein oder mehrere organische Verbindungen sind, ausgewählt aus der Gruppe, die aus Azobenzol, einem Diarylethen, einem Spiropyran, einem Spirooxazin, einem Fulgid und einem Leukochromophor besteht, und wobei zwei oder mehrere der Licht-reaktiven Moleküle in Form eines funktionellen Copolymers vorliegen können.

10. Zellkultiviervorrichtung nach Anspruch 1, wobei die Zelle eine Verankerungs-abhängige Zelle ist.

11. Verfahren zum Kultivieren und Sammeln einer Zelle, wobei das Verfahren folgendes umfasst:
(a) Anordnen einer Zelle in einer Zellkultiviervorrichtung, wobei die Vorrichtung eine Kulturschale mit einer Licht-reaktiven Zusammensetzung darauf umfasst, wobei die Licht-reaktive Zusammensetzung ein Licht-reaktives Material mit der Eigenschaft eines unterschiedlichen Haftvermögens für eine Zelle auf Grundlage der Menge der Lichtbestrahlung umfasst, gegenüber der die Licht-reaktive Zusammensetzung ausgesetzt wird,
(b) Kultivieren der Zelle in der Vorrichtung für eine ausgewählte Zeitspanne,
(c) Bestrahlen eines ausgewählten Bereichs der Licht-reaktiven Zusammensetzung mit Licht zur Freisetzung einer Zelle, die an der Licht-reaktiven Zusammensetzung in einer ausgewählten Region gebunden ist, und
(d) Sammeln der freigesetzten Zelle.

12. Verfahren nach Anspruch 11, wobei die Zelle eine Verankerungs-abhängige Zelle ist.

13. Verfahren nach Anspruch 11, wobei die ausgewählte Region der Licht-reaktiven Zusammensetzung die gesamte Region ist, die mit der Licht-reaktiven Zusammensetzung bedeckt ist.

14. Verfahren nach Anspruch 11, wobei das Verfahren weiterhin das physische Stimulieren der Zellkultiviervorrichtung oder die Zuführung eines Eluats zu dieser Zellkultiviervorrichtung oder beides, nach der Bestrahlung, umfasst, um die Freisetzung einer an der Licht-reaktiven Zusammensetzung gebundenen Zelle zu erleichtern.

15. Zellkultivier- und Sortiervorrichtung, wobei die Vorrichtung folgendes umfasst:
(a) Eine Zellkultiviervorrichtung, wobei die Vorrichtung eine Kulturschale mit einer Licht-reaktiven Zusammensetzung darauf umfasst, wobei die Licht-reaktive Zusammensetzung ein lichtempfindliches Material mit der Eigenschaft eines unterschiedlichen Haftvermögens für eine Zelle auf Grundlage der Menge der Lichtbestrahlung aufweist, gegenüber der die Licht-reaktive Zusammensetzung ausgesetzt wird;
(b) Ein Mittel zum Zuführen einer Kulturlösung in die Zellkultiviervorrichtung;
(c) Ein Mittel zur Bestimmung der Positionsinformation einer Zelle auf der Licht-reaktiven Zusammensetzung;
(d) Ein Mittel zur Bestrahlung einer ausgewählten Position der Licht-reaktiven Zusammensetzung der Zellkultiviervorrichtung mit Licht; und
(e) Ein Mittel zum Sortieren einer Zelle, die aus der Licht-reaktiven Zusammensetzung durch die Lichtbestrahlung freigesetzt worden ist.

16. Vorrichtung nach Anspruch 15, die weiterhin ein Mittel zum physischen Stimulieren der Zellkultiviervorrichtung oder ein Mittel zum Zuführen eines Eluats in die Zellkultiviervorrichtung, oder beides, einschließt.

17. Licht-reaktives Material, wobei das Material ein wärmeempfindliches Polymer umfasst, das sowohl hydrophile als auch hydrophobe Eigenschaften aufweist, die sich in Reaktion auf eine Temperatur verändern und wobei das Polymer durch einen photochromen Farbstoff modifiziert ist, wobei das Licht-reaktive Material eine unterschiedliche Klebekraft für eine Zelle zeigt, auf Grundlage der Menge der Lichtbestrahlung, der das Licht-reaktive Material ausgesetzt wird, so dass die Eigenschaft der unterschiedlichen Klebekraft dazu in der Lage ist, die Anlagerung und Ablösung einer Zelle, die auf einer Zellkultiviervorrichtung existent ist, zu regulieren.

18. Licht-reaktives Material nach Anspruch 17, wobei die Lichtbestrahlung durch Intensität, Dauer oder Wellenlänge des Lichtes oder eine Kombination dieser Faktoren definiert ist.

19. Zellkultiviervorrichtung, wobei die Vorrichtung eine Kulturschale mit dem Licht-reaktiven Material nach Anspruch 17 darauf umfasst.

## Revendications

1. Dispositif de culture cellulaire, ledit dispositif comprenant un récipient de culture ayant une composition photosensible dessus, dans lequel ladite composition photosensible comprend un matériau photosensible ayant une propriété d'adhérence différentielle pour une cellule basée sur une quantité d'irradiation lumineuse à laquelle ladite composition photosensible est exposée, dans lequel la propriété d'adhérence différentielle est capable de réguler la fixation et le détachement d'une cellule existant sur ledit dispositif.

2. Matériau photosensible selon la revendication 1, dans lequel l'irradiation lumineuse est définie par l'intensité, la durée ou la longueur d'onde de la lumière ou une combinaison de ces facteurs.

3. Dispositif de culture cellulaire selon la revendication 1, dans lequel ledit récipient de culture est un réceptacle adapté à la croissance d'une cellule constituée d'un composé sélectionné parmi le groupe consistant en du verre, du polystyrène, du chlorure de polyvinyle, du polyméthylpenthène, du polyéthylène téléphthalate, du polyéthylène et du polypropylène.

4. Dispositif de culture cellulaire selon la revendication 1, dans lequel ledit matériau photosensible sélectionné parmi le groupe consistant en (1) une composition comprenant un marqueur ou pigment et un polymère thermosensible ayant des propriétés hydrophiles-hydrophobes qui changent en réponse à un changement de température, (2) un matériau photocatalyseur, et (3) une molécule photosensible ayant la propriété d'adhérence différentielle pour une cellule basée sur une quantité d'irradiation lumineuse à laquelle elle est exposée.

5. Dispositif de culture cellulaire selon la revendication 4, dans lequel ledit marqueur ou pigment est un matériau qui absorbe la lumière et est sectionné parmi le groupe consistant en un marqueur organique, un pigment minéral et du carbone.

6. Dispositif de culture cellulaire selon la revendication 4, dans lequel ledit marqueur ou pigment est un matériau qui absorbe la lumière et est sélectionné parmi le groupe consistant en de l'indigo, une quinacridone, une porphyrine, du Fe₂O₃, HgS, CoO.nAl₂O₃ et du noir de carbone.

7. Dispositif de culture cellulaire selon la revendication 4, dans lequel ledit polymère thermosensible est sélectionné parmi le groupe consistant en un homopolymère et un copolymère, dans lequel ledit homopolymère et ledit copolymère consistent en un ou plusieurs parmi le N-isopropylacrylamide, le vinylméthyléther, le dimethylaminoethéylméthacrylate, un oxyethylènevinyléther, du styrenesulfonate de sodium et du chlorure de vinylbenzyltrimetylammonium.

8. Dispositif de culture cellulaire selon la revendication 4, dans lequel ledit matériau photocatalyseur est sélectionné parmi le groupe consistant en du TiO₂, VO₂, WO₃ et MoO₃.

9. Dispositif de culture cellulaire selon la revendication 4, dans lequel ladite molécule photosensible est un ou plusieurs composé(s) organique(s) sélectionné(s) parmi le groupe consistant en un azobenzène, un diaryléthène, un spiropyrane, une spirooxazine, un fulgide et un leuco chromophore, et dans lequel deux dites molécules photosensibles ou plus peuvent être sous la forme d'un copolymère fonctionnel.

10. Dispositif de culture cellulaire selon la revendication 1, dans lequel ladite cellule est une cellule dépendante d'ancrage.

11. Procédé pour cultiver et collecter une cellule, ledit procédé comprenant de :
(a) placer une cellule dans un dispositif de culture cellulaire, ledit dispositif comprenant un récipient de culture ayant une composition photosensible dessus, dans lequel ladite composition photosensible comprend un matériau photosensible ayant une propriété d'adhérence différentielle pour une cellule basée sur une quantité d'irradiation lumineuse à laquelle ladite composition photosensible est exposée,
(b) cultiver ladite cellule dans ledit dispositif pendant une période de temps sélectionné,
(c) irradier une région sélectionnée de la composition photosensible avec de la lumière pour libérer une cellule attachée à la composition photosensible au niveau de la région sélectionnée, et
(d) collecter ladite cellule libérée.

12. Procédé selon la revendication 11, dans lequel ladite cellule est une cellule dépendante d'ancrage.

13. Procédé selon la revendication 11, dans lequel ladite région sélectionnée de la composition photosensible est la région entière recouverte avec la composition photosensible.

14. Procédé selon la revendication 11, dans lequel ledit procédé comprend en outre de stimuler physiquement lesdits dispositifs de culture cellulaire ou d'alimenter un éluât dans ledit dispositif de culture cellulaire ou les deux après ladite irradiation pour aider à la libération d'une cellule fixée à la composition photosensible.

15. Appareil de culture et de triage cellulaire, ledit appareil comprenant :
(a) un dispositif de culture cellulaire, ledit dispositif comprenant un récipient de culture ayant une composition photosensible dessus, dans lequel ladite composition photosensible comprend un matériau photosensible ayant une propriété d'adhérence différentielle pour une cellule basée sur une quantité d'irradiation lumineuse à laquelle ladite composition photosensible est exposée ;
(b) des moyens pour alimenter une solution de culture dans ledit dispositif de culture cellulaire ;
(c) des moyens pour déterminer l'information de position d'une cellule sur ladite composition photosensible ;
(d) des moyens pour irradier une position sélectionnée de ladite composition photosensible dudit dispositif de culture cellulaire avec de la lumière ; et
(e) des moyens pour trier une cellule libérée de ladite composition photosensible par l'intermédiaire de ladite irradiation lumineuse.

16. Appareil selon la revendication 15, qui comporte en outre des moyens pour stimuler physiquement ledit dispositif de culture cellulaire ou des moyens pour alimenter un éluât dans ledit dispositif de culture cellulaire ou les deux.

17. Matériau photosensible dans lequel ledit matériau comprend un polymère thermosensible ayant des propriétés à la fois hydrophiles et hydrophobes qui changent en réponse à un changement de température et dans lequel ledit polymère est modifié par un marqueur photochrome, dans lequel ledit matériau photosensible montre une adhérence différentielle pour une cellule basée sur une quantité d'irradiation lumineuse à laquelle ledit matériau photosensible est exposée de sorte que la propriété d'adhérence différentielle est capable de réguler la fixation et le détachement d'une cellule existant sur un dispositif de culture cellulaire.

18. Matériau photosensible selon la revendication 17, dans lequel l'irradiation lumineuse est définie par l'intensité, la durée ou la longueur d'onde de la lumière, ou une combinaison de ces facteurs.

19. Dispositif de culture cellulaire, ledit dispositif comprenant un récipient de culture ayant le matériau photosensible selon la revendication 17 dessus.
